# EUROPEAN PATENT SPECIFICATION

(11) **EP 1 699 501 B1**
(45) Date of publication and mention of the grant of the patent: **08.02.2012**
(21) Application number: 04812694.0
(22) Date of filing: 02.12.2004
(51) Int. Cl.: A61L 29/14, A61L 29/16, A61L 27/38, A61L 27/50, A61F 2/06, A61F 2/24, A61M 25/00

(54) **KITS FOR MAGNETICALLY COATING MEDICAL DEVICES WITH LIVING CELLS**
SETS FÜR DIE MAGNETISCHE BESCHICHTUNG VON MEDIZINPRODUKTEN MIT LEBENDEN ZELLEN
TROUSSES POUR L'ENROBAGE MAGNETIQUE DE DISPOSITIFS MEDICAUX A L'AIDE DE CELLULES VIVANTES

(30) Priority: 03.12.2003 US 526522 P
(43) Date of publication of application: 13.09.2006
(73) Proprietor: MAYO FOUNDATION FOR MEDICAL EDUCATION AND RESEARCH, Rochester, MN 55905 (US)
(72) Inventor: SANDHU, Gurpreet, S., Rochester, MN 55906 (US); SIMARI, Robert, D., Rochester, MN 55902 (US); SANDHU, Nicole, P., Rochester, MN 55906 (US); GULATI, Rajiv, Rochester, MN 55902 (US)
(74) Representative: Vossius & Partner
(86) International application number: PCT/US2004/040242
(87) International publication number: WO 2005/056073

(56) References cited:
- EP-A- 0 884 064
- WO-A-01/93939
- WO-A-03/037400
- US-A- 5 851 218

## Description

A recurring issue in the field of medical devices, especially implantable medical devices, is the need to provide surfaces that are compatible with the environment in which they are contained.

Problems associated with implanted medical devices that include surfaces in contact with a patient's bloodstream may include, for example, the risk of acute thrombosis and chronic instability--such as calcification--of the implant surface. Surfaces of, e.g., prostheses that are implanted as part of the circulatory system can be a crucial factor governing the functionality and patency rates of the synthetic prostheses. Poor blood compatibility of these surfaces is almost always a predominant reason for the limitations of these implants, such as the loss of heart valve functionality over time or poor patency rates in small diameter conduits due to acute thrombosis or intimal hyperplasia. Attempts to modify the surfaces of synthetic grafts to overcome the patency problems associated with thrombosis or intimal hyperplasia have generally shown poor long-term outcomes, as these surfaces are unable to maintain a sustained anti-thrombogenic bioactivity as discussed in, e.g., U.S. Patent Application Publication No. US 2003/0082148 A1 (Ludwig et al.).

One surface modification approach which has been utilized for blood contacting implants such as synthetic grafts is "endothelial seeding". *In vitro* endothelial seeding utilizes viable endothelial cells which are seeded onto the blood contacting surface of a prosthesis such as the lumen surface of a vascular graft to mimic the surface of natural blood vessels. This surface modification technique aims to produce a confluent, biologically active surface of viable endothelial cells which by definition, is anti-thrombogenic.

Problems arise, however, in the retention of suitable cells on the surfaces of the devices. Techniques aimed to improve the retention of endothelial cells on vascular grafts are described in, e.g., U.S. Patent No. 5,037,378 (Muller et al.). In another approach (described in, e.g.. U.S. Patent No. 4,804,382 (Turina et al.)), endothelial cells are applied to a semi-permeable membrane in which the pores are filled with aqueous gels to allow endothelial cell coverage. Another approach to prevent endothelial loss after seeding is to modify the graft lumen surface to make it sufficiently adhesive for endothelial cells. Surface modification methods include the interstitial deposition of protein glues or matrices, the adsorption of proteins to the graft surface, and the covalent immobilization of adhesion-promoting ligands, peptides, or proteins onto functional groups introduced by chemical modification or gas plasma treatment. Another method for preventing the loss of endothelial cells and for improving patency rates of synthetic grafts involves using shear stress to precondition the endothelial layer of a synthetic graft.

Still another set of methods for retaining endothelial and other cells on graft surfaces are described in U.S. Patent Application Publication No. US 2003/0082148 A1 (Ludwig et al.). One set of techniques disclosed in that reference involves, e.g., the use of ligands for *in vivo* recruitment of target cells, where the ligands include molecules that are binding partners to a molecule presented on the surfaces of the target cells. Another set of techniques involves *in vivo* recruitment of target cells using magnetic forces, where the target cells are magnetically charged and released within the bloodstream of the patient. As the magnetically charged target cells pass near magnetic graft, they are captured on the surface.

Unfortunately, however, *in vivo* recruitment techniques suffer from a number of disadvantages. One significant disadvantage is the inability to determine whether the magnetic forces exhibited by the surfaces are sufficient to attract target cells before implantation. A failure to properly magnetize a device including magnetic material that is not permanently magnetic may result in less than optimal *in vivo* recruitment.

Another potential disadvantage is the time required to recruit significant numbers of target cells to the desired surfaces. Days or weeks may be required to adequately cover the exposed surfaces of the device. Furthermore, even after significant periods of time, the target cells may be attached at low density over the surfaces, resulting in less than optimal performance. If the surfaces are located in areas of limited or no blood flow, then recruitment may be further hampered or prevented.

### SUMMARY OF THE INVENTION

The present invention as defined in the claims provides a bit for magnetically coating medical devices with surfaces on which viable biologic cells are magnetically attracted and retained. Methods involve attraction and retention of magnetic cells on magnetic contact surfaces of medical devices.

One potential advantage of the present invention is the ability to rapidly attract and attach large numbers of magnetic cells to the magnetic contact surfaces of a medical device. This is possible because the device can be located in a carrier liquid containing high concentrations of magnetic cells before or after implantation. The carrier liquid with magnetic cells may be in contact with the medical device *in vitro* or *in vivo.* In either case, it may be preferred that the carrier liquid have a concentration of magnetic cells that is high enough to facilitate coating of the medical device within an acceptable period of time, e.g., several hours or less.

If the magnetic coating is performed *in vivo,* it may be preferred that the carrier liquid with magnetic cells be delivered to a defined volume in which the medical device is located. If the medical device is implanted within, e.g., a blood vessel, the defined volume may be created by using a catheter to seal the vessel on one or both sides of the implanted medical device, followed by delivery of the carrier liquid to the location of the implanted medical device.

Another potential advantage of the present invention is that the medical devices may be located in any suitable location within the body and coated with magnetic cells after implantation, i.e., the medical devices and methods may be used in blood vessels, but the potential locations are not limited to blood vessels. For example, the medical devices may be located and magnetically coated within the gastrointestinal tract, salivary glands, bile ducts, pancreatic ducts, renal system (kidney, ureters, bladder, urethra, etc.), airways in lungs, conduits in the brain and spinal cord, surgically created conduits or lumens in an organ, congenital abnormalities in any organ, etc.

Another potential advantage of the present invention is that medical devices constructed of metals and/or metal alloys may be easily and quickly magnetized by exposure to a magnetic field (if the metals or metal alloys do not inherently exhibit magnetic fields). In some embodiments, the apparatus and methods disclosed provide the ability to magnetize a medical device as part of the implantation process or to magnetize the medical device *in vivo.* In some instances, it may be possible to use known medical devices such as, e.g., stents, heart valves, etc. in connection with the present invention.

Yet another potential advantage is the ability to demagnetize an implanted medical device after magnetically coating the medical device by, e.g., locating a degaussing element proximate the medical device *in vivo* after a selected period of time, e.g., 24 hours to 48 hours. By magnetically coating the medical device, the advantages associated with magnetic coating as discussed herein may be obtained and any potential disadvantages associated with leaving a magnetized medical device may be alleviated by demagnetizing the medical device after the magnetically attracted biologic cells have formed alternative attachments to the medical device.

Still another potential advantage of some embodiments that cells can be magnetically attracted to and retained on the medical devices without requiring additional modifications for cellular attachments such as receptor-ligand binding, antigen-antibody interactions, or other coatings (such as, e.g., fibronectin) to obtain adequate cell adhesion. Examples of some such binding techniques that may be avoided by use of the present invention include, but are not limited to, e.g., ligand binding, fibronectin, antibodies, proteins, etc.

Another potential advantage is that, in some embodiments, all of the magnetic contact surfaces of the medical devices can be coated by the magnetic cells. The ability to magnetically attach cells to all magnetic surfaces may provide improved coverage over all surfaces, even those located in areas not otherwise easily accessible.

Still another potential advantage may, in some embodiments, be found in the ability to effectively monitor attraction and retention of the magnetic cells on the magnetic contact surfaces of the medical devices. Such monitoring may preferably be performed before using the medical device (e.g., implanting it in a patient). If the cells are unevenly or insufficiently retained on the medical device, steps can be taken to improve attraction and/or retention before the device is implanted or to simply discard the device.

The present invention provides a kit for magnetically coating a medical device *in vivo,* the kit including an implantable medical device having a magnetic contact surface; and means for enclosing a defined volume *in vivo* in which the implantable medical device is located when implanted.

In another aspect, the present invention provides a kit for magnetically coating a medical device *in vivo.* The kit includes an implantable medical device with a magnetic contact surface and a catheter for creating a defined volume in a biologic conduit *in vivo.* The catheter may include at least one seal capable of closing the biologic conduit when deployed within the conduit; a carrier liquid lumen extending from a proximal end of the catheter to a location proximate the at least one seal; and an opening in the carrier liquid lumen proximate the at least one seal, wherein carrier liquid in the carrier liquid lumen is capable of exiting the catheter proximate the seal.

In another aspect, the disclosure relates to a method of magnetically coating an implanted medical device *in vivo* by providing a carrier liquid with a plurality of magnetic cells, wherein each cell of the plurality of magnetic cells is a viable biologic cell. The method further includes contacting the implanted medical device with the carrier liquid *in vivo,* wherein the magnetic cells are present in the carrier liquid at a concentration of 1000 cells per milliliter or higher.

In another aspect, the disclosure relates to a method of magnetically attaching cells to an implantable medical device by locating an implantable medical device in a carrier liquid containing a plurality of magnetic cells *in vitro,* wherein each cell of the plurality of magnetic cells is a viable biologic cell, and wherein a plurality of the plurality of magnetic cells are magnetically attracted to and retained on the implantable medical device. The method further includes implanting the implantable medical device in a body after the plurality of magnetic cells are magnetically attracted to and retained on the implantable medical device.

These and other features and advantages of the present invention may be described in more detail below with respect to some illustrative embodiments of the invention.

### BRIEF DESCRIPTION OF THE DRAWINGS

FIG. 1 is a perspective view of one exemplary medical device according to the present invention.
FIG. 2 is a schematic diagram of one method for magnetically coating an implanted medical device *in vivo.*
FIG. 3 is a schematic diagram of one apparatus and method for magnetizing an implanted medical device *in vivo.*
FIG. 4 is a schematic diagram of one apparatus and method for magnetizing an implantable medical device during the implantation process.

### DETAILED DESCRIPTION OF ILLUSTRATIVE EMBODIMENTS

In the following detailed description of illustrative embodiments, reference is made to the accompanying drawings that form a part hereof, and in which are shown, by way of illustration, specific embodiments in which the invention may be practiced.

Furthermore, like reference numbers denote like features in the different figures.

It should be noted that as used herein and in the appended claims, the singular forms "a", "and", and "the" include plural referents unless the context clearly dictates otherwise. Thus, for example, reference to "a magnetic cell" includes a plurality of such cells and reference to "the magnetic contact surface" includes reference to one or more magnetic contact surfaces and equivalents thereof known to those skilled in the art.

### MEDICAL DEVICES WITH MAGNETIC CONTACT SURFACES

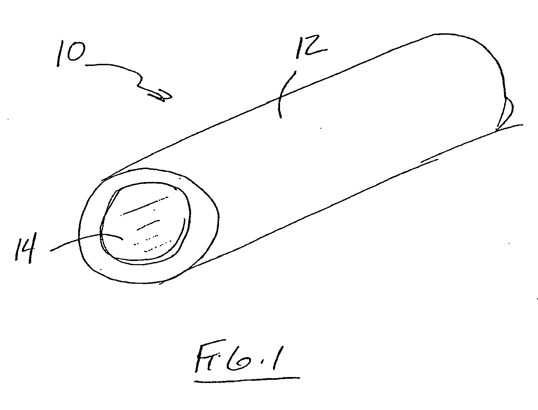

FIG. 1 depicts one illustrative embodiment of a medical device 10 that can be used in connection with the present invention. The depicted device 10 includes an exterior surface 12 and an interior surface 14 that defines, at least in part, a lumen or passageway formed through the device 10. The device 10 may be broadly described as a stent and, although depicted as a solid tubular object, it will be understood that the device 10 may be constructed as a slotted metal tube, wire mesh structure, etc. If provided in the form of a stent as shown, the device 10 may be used to prop open a blood vessel or other tubular structure such as, e.g., bile duct, ureter, etc. The stent may further be designed to open under force using, e.g., an inflatable balloon, or, alternatively, to be self-expanding as is known in the art. Examples of only a few stent designs can be seen in, e.g., U.S. Patent Nos. 4,733,665 (Palmaz), 4,503,569 (Dotter), etc.

Although described herein in connection with a medical device 10 in the form of a stent as depicted in FIG. 1, it should be understood that the medical devices may include any medical device that comes in contact with biological material of a living body. In one broad class of medical devices, may find use with implantable medical devices, i.e., medical devices designed for implantation in an animal or human body. Examples of some suitable implantable medical devices include, but are not limited to, stents, synthetic vascular grafts, prostheses for other biologic conduits (e.g., neurologic, gastrointestinal, renal, endocrine, pulmonary, urologic, etc.), heart valves, artificial hearts, left ventricular assist devices, coronary device leads (for, e.g., pacemakers, defibrillators, etc.), arterio-veinous fistulas for dialysis, etc.

In other applications, coils used to embolize and occlude aneurysmal sacs in blood vessels or to occlude blood vessels supplying malignant tumors may be magnetically coated with cells to expedite healing in aneurysms or the occlusion of vessels to tumors. Devices for treating endovascular leaks (holes/ruptures in blood vessels, valves or heart chambers) may also be magnetically coated with cells to facilitate healing and reduce thrombosis. Magnetic devices may also be used to localize cells for treating malignancies in, e.g., organs. Magnetic devices and cells (that, e.g., produce therapeutic agents) may also be placed in blood vessels that supply a tumor.

Furthermore, the medical devices may be used in *ex vivo* applications where surfaces of the devices come into contact with biologic materials (e.g., blood) outside of the body of the subject providing the biologic fluids. Examples of such medical devices may include, e.g., artificial organs, blood pumps, etc.

Both the exterior surfaces 12 and the internal surfaces 14 of the medical device 10 may be considered "contact surfaces" in connection with the present invention where the device. The term "contact surfaces," for the purposes of the present invention, means any surface that can contact a carrier liquid in which the medical device is located for coating. For example, if the device 10 is a stent, any intermediate surfaces of wires, mesh structures, etc. that may be used to construct the device 10 and that extend between what might be defined as the exterior surface 12 and the interior surface 14 may also be considered "contact surfaces" provided those surfaces are exposed to and can contact a carrier liquid in which the medical device 10 is placed during the coating process.

It may be preferred that all of the contact surfaces of the medical devices may exhibit magnetic fields such that they can magnetically attract and retain objects such as cells using magnetic forces. Contact surfaces of medical devices that exhibit magnetic fields are described as "magnetic contact surfaces." In some instances, it may be preferable to provide a medical device in which only some or portions of some of the contact surfaces on the medical device exhibit magnetic fields (i.e., are magnetic contact surfaces).

The medical devices may be made of any suitable material (including, e.g., polymeric materials, metals, metal alloys, ceramics, composites, etc.) provided that the contact surfaces of the medical devices that are to be magnetically coated exhibit magnetic fields capable of attracting and retaining magnetic cells as described herein. To exhibit magnetic fields, the medical devices preferably include one or more materials that are magnetic, i.e., that either exhibit a permanent magnetic field or that are capable of exhibiting a temporary magnetic field.

The entire medical device 10, or selected portions thereof, may be manufactured from the one or more magnetic materials. For example, a predetermined quantity of magnetite or an alloy thereof may be included in the construction of the device 10. Other materials may be utilized in addition to or in place of magnetite to provide the desired magnetic properties. Such materials may be temporary magnetic materials or permanent magnetic materials. Some examples of suitable magnetic materials include, e.g., magnetic ferrite or "ferrite" which is a substance consisting of mixed oxides of iron and one or more other metals, e.g., nanocrystalline cobalt ferrite. However, other ferrite materials may be used.

Other magnetic materials which may be utilized in the construction of the device 10 may include, but are not limited to, ceramic and flexible magnetic materials made from strontium ferrous oxide which may be combined with a polymeric substance (such as, e.g., plastic, rubber, etc.); NdFeB (this magnetic material may also include Dysprosium); neodymium boride; SmCo (samarium cobalt); and combinations of aluminum, nickel, cobalt, copper, iron, titanium, etc.; as well as other materials.

Where the device 10 is made of metals such as, e.g., stainless steel, nickel titanium alloys (e.g., NITINOL), etc. or other magnetizable materials, the magnetic contact surfaces of the device may be rendered sufficiently magnetic by subjecting the magnetizable material to a sufficient electric and/or magnetic field. Such a field may imbue the magnetic contact surfaces (or a portion thereof) with magnetic properties without the need to include the permanent magnetic materials described above.

In some embodiments, the magnetic coating surfaces of the medical devices to be coated with magnetic cells through magnetic attraction and retention may consist essentially of one or more metals and/or metal alloys, e.g., stainless steel, nickel titanium alloy, etc. In another manner of characterizing the present invention, the magnetic contact surfaces of the medical device may consist essentially of one or more magnetic materials, where the magnetic materials are permanently magnetic or not as described herein. The magnetic contact surfaces of the device may include one or more magnetic materials dispersed in a non-metallic binder.

It may be preferred that medical devices include a magnetizable coating such as, e.g., nickel or other materials with a similar susceptibility to magnetization, regardless of whether the body of the medical device includes magnetic or magnetizable materials. For example, it may be preferred that the body of the medical device (e.g., a stent) be constructed of stainless steel that is plated with nickel to increase the magnetic field exhibited by the medical device after magnetization.

If the medical device 10 is designed to be implanted within a human or animal body, its contact surfaces are preferably biocompatible. Unfortunately, many magnetic materials may not be biocompatible. The non-biocompatible magnetic materials may preferably be coated with a biocompatible material that does not significantly limit or interfere with the magnetic fields emanating from the magnetic materials in the medical device such that the magnetic contact surfaces exhibit the desired magnetic fields in the presence of the coating. Biocompatible coatings for use as magnetic contact surfaces on medical devices include various biocompatible polymers, metals, and other synthetic, natural, or biologic materials.

In some instances, it may be desirable to provide magnetic contact surfaces that, in addition to exhibiting a magnetic field capable of attracting and retaining a magnetic cell, may also include materials that provide for molecular binding in addition to magnetic attraction and retention. Such molecular binding may be targeted to the same cells as the magnetic cells or to different cells, i.e., cells other than those that are magnetized. For example, the magnetic contact surfaces may also be functionalized to allow for the attachment of cell binding ligands, as well as other molecules such as, e.g., cell mobilization enhancer molecules, molecules for cellular retention and spreading, molecules for cell differentiation, pharmaceutical compounds, etc. In some medical devices it may be desirable to functionalize surfaces of the medical device that are not magnetic contact surfaces whether or not the magnetic contact surfaces are themselves functionalized.

The functionalization of a surface may involve, e.g., gas plasma treatment, chemical modification, photochemical modification, chemical modification through y-radiation activation, co-polymerization with molecules containing functional groups, as well as other surface modification techniques known in the art. The surface to be modified may, for example, be subject to ozonolysis to introduce carbonyl or other reactive groups thereon that will facilitate the attachment of ligands of interest to the chosen surface. In other instances, the surface may be subjected to treatment with acid or base solutions to form hydroxyl and/or carboxylic acid functionalities thereon. Functionalization of the surface to be modified may also be achieved by coating the surface with a layer of polymeric material having a desired functionality. Such polymer layers may include, for example, polyamines such as poly(L-lysine) and poly(L-glutamine) to provide amine functionalities on the specified surface.

### MAGNETIC CELLS

The present disclosure includes devices with magnetic contact surfaces that are coated with magnetic cells attracted to and retained on the magnetic contact surfaces by magnetic forces. The present disclosure also includes methods of coating the magnetic contact surfaces with magnetic cells.

The cells that are used as magnetic cells in connection with the present invention may include, e.g., any biologic cell that is capable of itself exhibiting a magnetic charge, being modified to incorporate one or more particles that include a magnetic charge, or that can be attached to a particle or cell that includes a magnetic charge. The magnetic cells may be, e.g., endothelial cells, when used in blood contacting medical devices. In addition, the magnetic cells may be, e.g., ectoderm-, mesoderm-, endoderm- derived cells. Additionally, any stem or mature cell originating from various primitive cell layers in animals or humans may be modified to become magnetic cells useful in connection with the present disclosure.

In other variations, the magnetic cells may be engineered to carry new genes that may secrete products capable of treating disease, e.g., heart failure, coronary artery disease, cancer, etc.

A variety of techniques for modification of cells such that the cells become magnetic cells subject to magnetic attraction are known. Magnetic particles may be incorporated into the cell or attached to the cell surface by procedures known to those skilled in the art. In certain embodiments, magnetic particles may be fed to the target cells (Moller W, et al. (1997) J Aerosol Med 10:173-186; Violante (1990) Acta Radiol Suppl 374: 153-156) or temporary pores may be created in the cell membrane of the target cell by electroporation (Moroz & Nelson (1997) Biophys J 72:2211-6; Zhelev & Needham (1993) Biochim Biophys Acta. 1147(1):89-104; Neumann E, Kakorin S, Toensing K. (1998) Faraday Discussions 111: 111-125). In other embodiments, magnetic particles may be attached to the cell surface via antibody binding to cell membrane receptors or through chemical conjugation of the magnetic particle to the cell membrane (Yin, A H; Miraglia, S; Zanjani, E D; Almeida-Porada, G; Ogawa, M; Leary, A G; Olweus, J; Kearney, J; Buck, D W (1997) Blood 90: 5002-5012; Buckley et al. ABL 1998 June 30-32).

In certain embodiments, cells may be magnetically modified or labeled by intravenous injection of magnetic particles which are conjugated to molecules which in turn will attach to the surface of the cells to be recruited to the surface. One such example constitutes the antibody-mediated binding of magnetic particles to the CD133 or CD34 protein found on the surface of several progenitor cell types. These cells may be endothelial progenitor cells or mature endothelial cells which may or may not have been genetically modified to express or produce an agent with an inhibitive effect on smooth muscle cell proliferation.

Para- or ferromagnetic particles may be enclosed in lipid membrane vesicles (liposomes) associated with the targeted cell or within a polymer matrix of micro- and nanoparticles attached to the cell of interest. Alternatively, the magnetic particles may be conjugated to the cellular surface of the targeted cell to constitute part of the cellular membrane. The cells may be recruited from the bloodstream to the magnetically charged prosthesis by magnetic attraction.

The strength of magnetic attraction typically depends on the magnetic properties of the particles utilized to modify the cells to be recruited to the magnetic contact surface, as well as the strength of the magnetic field emanating from the magnetic contact surface, and the gradient of this field where both the field and its gradient will vary with location. The magnetic properties of the particle depend on the chemical composition of the particle as well as its magnetization state. The properties of the magnetic field depend on surface and body geometry, the chemical composition and magnetic history of the device. Once attracted to the magnetic contact surface, the cells are preferably retained on the surface and, in certain embodiments, cell adhesion may induce cellular spreading and differentiation over the magnetic contact surface(s) as well as other surfaces of the medical device.

In other embodiments, increasing cell affinity to magnetic contact surfaces may include incorporating magnetic particles into the cells through fusion of vesicles to the cells. A vesicle defines a volume enclosed by a membrane. This membrane may include proteins, lipids, polymers, blockcopolymers, or a mixture thereof. When such a vesicle fuses with a cell, the vesicle volume becomes part of the cell plasma and the vesicle's contents are released into the cell interior. If the vesicle is loaded with magnetic particles during vesicle formation, fusion of the vesicles with the cells results in incorporation of these magnetic particles into the cells' interiors.

Another technique for incorporating magnetically sensitive particles into target cells is by endocytosis. For this purpose, magnetic particles are fed to cells with endocytotic capabilities. Upon contact with a particle, cells, which may be stimulated to do so, will engulf the particle by adhering their membrane to the particle, followed by increasing the area of adherence until the entire particle is enclosed by a membrane section of the cell. After enclosure, the particle is incorporated into the cell interior by virtue of invagination of the membrane enclosed particle. In yet another embodiment, small magnetic particles with a diameter between, e.g., 50 nanometers (nm) and 250 nm may be brought into the target cell by creating temporary pores in the cell membrane through electric field exposure (i.e. electroporation). These standard techniques and others useful in the methods of the invention may be described by the following references, Moller W, Takenaka S, Rust M, Stahlhofen W, Heyder J. (1997); J Aerosol Med 10:173-186; Violante (1990) Acta Radiol Suppl 374: 153-156); Moroz & Nelson (1997) Biophys J. 72:2211-6; Zhelev & Needham (1993) Biochim Biophys Acta. 1147(1):89-104.

Modification of cell magnetic properties in certain embodiments may include attaching magnetically sensitive particles, such as ferromagnetic or paramagnetic particles (including, but not limited to, e.g., ferrite, samarium cobalt, neodymium boron) to the surface of the cells. This may be achieved by modifying the surface of these particles to have affinity for the membrane of the cell. This affinity may be established by attaching ligand molecules to the magnetic particle. The magnetic particle can then bind via the ligand to an appropriate cell surface molecule present on the outer surface of the cell. The binding of magnetic particles to the cell membrane may also be achieved by reacting the magnetic particle (or a particle-encapsulating polymer matrix) to molecular groups typically found at cell membranes, including groups such as amine or thiol or hydroxyl groups, through chemically reactive groups presented at the particle or matrix surface.

In other embodiments, the cell can be modified to be magnetically charged by encapsulating the magnetic particle within or attached onto a polymeric matrix that is modified to have an affinity to the cell membrane. The surface of magnetic particle and/or the polymeric matrix may include proteins or peptide sequences, e.g., such as RGD peptides, which provide sites of attachment for cell surface integrins.

Standard protocols (as described by, e.g., Kemshead J T, Ugelstad J. (1985) Mol Cell Biochem 67: 11-18) have been utilized to magnetically modify target cells of the invention. The size of these particles is dependent on target cell type as well as the desired strength of the magnetic attraction. Magnetic particles that may be useful may have a diameter which ranges from 50 nm or higher, in some instances 100 nm or higher. At the upper end of the range, the magnetic particles may have a diameter of 5 micrometers (µm) or less, or in some instances 1 µm or less.

### COATING MAGNETIC CONTACT SURFACES WITH MAGNETIC CELLS

The methods involve magnetically attracting and retaining magnetic cells on magnetic contact surfaces of medical devices. The coating is preferably accomplished by locating the medical device in a carrier liquid containing the magnetic cells. The magnetic contact surfaces of the medical device are located within the carrier liquid such that the magnetic cells within the carrier liquid can be magnetically attracted to and retained on the magnetic contact surfaces.

The coating process be accomplished *in vivo* or *ex vivo* (e.g., *in vitro*), wherein the magnetic contact surfaces of the medical device can be located within a carrier liquid containing concentrations of magnetic cells that are significantly higher than, e.g., the concentration of magnetic cells in the bloodstream in an *in vivo* recruitment process such as that described in, e.g., U.S. Patent Application Publication No. US 2003/0082148 A1 (Ludwig et al.).

The carrier liquid in which the magnetic cells are provided may take a variety of forms, although it may preferably be a biologically compatible liquid. Examples of some suitable biologically compatible carrier liquids include, but are not limited to, solutions of 0.9% normal saline, Ringer's solution, various tissue culture media, serum, plasma, whole blood, etc. The solutions may also include one or more of, e.g., growth factors, hormones and other biologic or synthetic materials.

Regardless of the composition of the carrier liquid, the concentration of magnetic cells in the carrier liquid may preferably be significant. For example, the concentration of magnetic cells may be, e.g., 1000 magnetic cells per milliliter (ml) or higher, in some instances 10,000/ml or higher, and in still other instances 100,000/ml or higher, or even 1,000,000/ml or higher.

It may be preferred, in some embodiments, that the magnetic cells that are magnetically attracted to and retained on the implantable medical device are retained on the implantable medical device in the absence of ligand binding, fibronectin, antibodies, proteins, etc. Essentially, any biocompatible device surface may be used for direct cell to device surface contact without requiring surface modification. In other words, it may be preferred that magnetic force is the primary force involved in both attracting and retaining the magnetic cells on the magnetic contact surfaces of the medical devices.

In other embodiments, the methods may be characterized on the basis of the time required to magnetically coat the medical devices. For example, the methods may involve locating the medical device to be coated in the carrier liquid containing the magnetic cells for a period of one hour or less to coat the magnetic coating surfaces to a desired level. In some instances, the coating may be accomplished in less time, for example, in some instances, it may be possible to coat the magnetic coating surfaces by locating the medical device in the carrier liquid for a period of 30 minutes or less, 15 minutes or less, 10 minutes or less, etc.

The methods may also be characterized on the basis of coverage of the magnetic contact surfaces of the medical devices (for either *in vivo* or *in vitro* coating methods). For example, it may be preferred that, for the magnetic contact surface of the medical device in contact with the carrier liquid containing the magnetic cells, 25% or more of the magnetic contact surface in contact with the carrier liquid is coated by the magnetic cells. In some cases, it may be preferred that 50% or more of the magnetic contact surface located in the carrier liquid be coated by the magnetic cells, and in other cases it may be preferred that substantially all of the magnetic contact surfaces located in the carrier liquid be coated by the magnetic cells. In addition, these coating levels, e.g., coating 25% or more of the magnetic contact surface in contact with the carrier liquid with the magnetic cells may preferably be performed within time periods of 30 minutes or less, 15 minutes or less, or even 10 minutes or less.

The coating levels described in the preceding paragraph may, in some instances, be obtained before the medical device is used, e.g., implanted in a body. Furthermore, the methods may include inspection of the magnetic contact surfaces to determine if the desired coating level is achieved before using (e.g., implanting, etc.) the medical device. If the inspection reveals that the coating is not acceptable, the medical device can be returned to a carrier liquid containing the desired magnetic cells to improve the coating level.

In some embodiments, the methods may involve coating only a portion of the magnetic contact surfaces of the medical device with magnetic cells. Such selective coating of the magnetic surfaces may be performed by, e.g., contacting only selected portions of the magnetic contact surfaces with the carrier liquid containing the magnetic cells.

The methods may, in some instances, involve contacting all of the magnetic contact surfaces on a medical device with the carrier liquid containing the magnetic cells, such that, for example, the magnetic cells can be magnetically attracted to and retained on all of the magnetic contact surfaces. Such a method, may, for example, involve immersing the entire medical device in the carrier liquid containing the magnetic cells.

Because the medical devices may be constructed of materials that are not permanently magnetic, the methods may, in some instances, involve magnetizing at least a portion of the medical device to form the magnetic contact surfaces that will be coated when the medical device is located in a carrier liquid containing the magnetic cells. Magnetic fields may be induced by any suitable technique, e.g., locating the medical device in an electric field, locating the medical device in a magnetic field, etc.

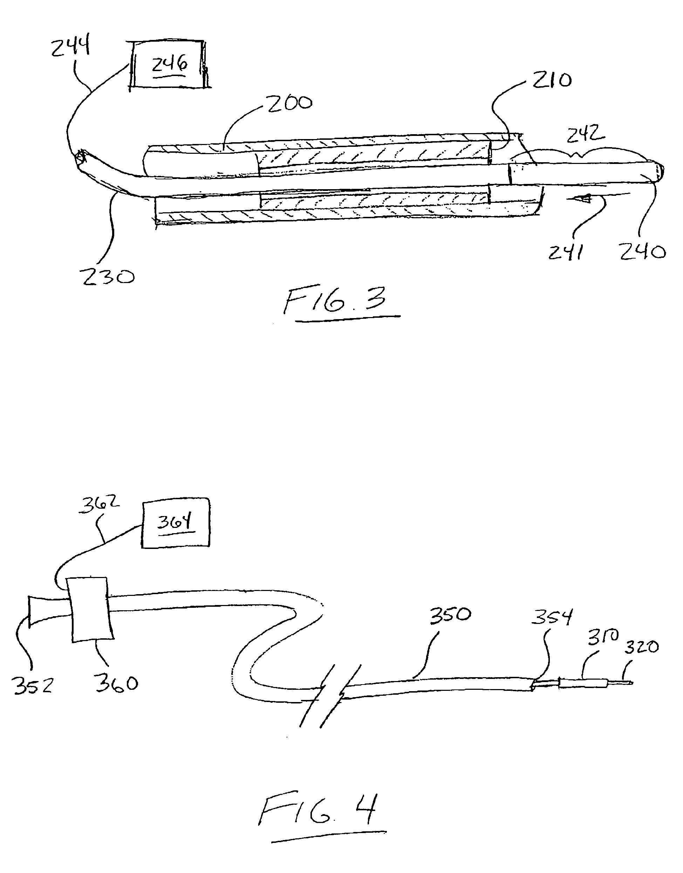

One potential apparatus and method of magnetizing a magnetizable medical device is depicted in FIG. 3. The medical device 210 is in the form of a stent deployed within a vessel 200. The medical device 210 preferably includes one or more magnetizable materials such that one or more magnetic contact surfaces can be provided on the medical device 210.

If the magnetizable materials do not inherently exhibit magnetic fields of sufficient strength to attract magnetic biologic cells as discussed herein, it may be advantageous to expose the magnetizable materials to a magnetic field or electric field after implantation within a subject. The depicted apparatus includes a medical grade catheter 230 that includes a magnetic field generator 240 adapted for deployment through the lumen formed within the stent 210. In other embodiments, the medical device may or may not include a lumen through which the magnetic field generator can pass. In such embodiments, it may be sufficient that the magnetic field generator pass near the implanted medical device.

As used herein, a "medical grade catheter" is a catheter that is constructed such that it may be inserted into the body of a subject. Preferably, a medical grade catheter is capable of being sterilized. The magnetic field generator 240 may, e.g., occupy a section 242 of the catheter 230 proximate the distal end thereof as seen in FIG. 3.

It is typically preferred that the magnetic field generator 240 provide a magnetic and/or electric field sufficient to induce the magnetic materials within the medical device 210 to exhibit a magnetic field (if they are not permanently magnetized). The induced magnetism in the medical device 210 is preferably strong enough to magnetically attract biologic cells as discussed herein when the magnetic field generator 240 is advanced through the medical device 210 and/or drawn back through the medical device 210 in, e.g., the direction of the arrow 241. In some embodiments, the magnetic field generator may preferably reside in or near the medical device 210 for a selected dwell time.

In some embodiments, the magnetic field generator 240 may be in the form of one or more magnets. In other embodiments, the magnetic field generator may be in the form of one or more electric coils connected to a power source 246 by an electrical lead 244 extending along the length of the catheter 230 and providing electrical energy to the coil. The power source 246 may preferably be located outside of the body of the subject in which the medical device 210 is implanted. In either form, the magnetic field generator 240 is preferably capable of inducing magnetization in magnetizable materials located in close proximity to the magnetic field generator 240.

The catheter 230 and its magnetic field generator 240 may also serve a function other than magnetizing the medical device 210. In some embodiments, the catheter 230 may be used to degauss or demagnetize an implanted medical device 210. Demagnetization may be desired after the magnetically attracted biologic cells have formed other attachments to the medical device 210 (e.g., after 24-48 hours). If the magnetic field generator 240 is configured as a degaussing element to degauss a magnetic medical device, then *in vivo* degaussing of a medical device may be performed.

If degaussing is performed as a part of the present invention, it should be understood that the degaussing may not necessarily eliminate the magnetic fields exhibited by a medical device. Rather, the degaussing may simply reduce the magnetic field strength below the magnetic field strength exhibited by the magnetic contact surfaces before the degaussing. In some instances, it may be desirable that the magnetic field strength be reduced as a result of the degaussing by, e.g., 50% or more.

It should be understood that although the catheter 230 and its magnetic field generator 240 are depicted in connection with a medical device 210 in the form of a stent, the *in vivo* magnetization of an implanted medical device may be performed with virtually any implanted medical device. Furthermore, the magnetic field generator need not be inserted through an implanted medical device. Rather, the magnetic field generator need only be located close enough to the implanted medical device to induce the desired magnetization in the medical device.

In some instances, the magnetic field generator may not even need to be advanced to an *in vivo* location to magnetize an implanted medical device. For example, a magnetic field generator may be located outside of the subject's body in a location near enough to the implanted medical device to induce magnetization of the magnetic materials in the medical device. In such an application, degaussing may also be performed using an external degaussing unit located proximate the implanted medical device.

The apparatus depicted in FIG. 4 is one example of another technique for magnetizing a medical device for *in vivo* magnetic coating. The apparatus includes an elongated body 350 such as, e.g., a guide catheter, sheath, etc. that preferably includes at least one lumen extending from a proximal end 352 to a distal end 354. The body 350 may preferably be adapted to assist in the delivery of a medical device 310 to an internal body location. The medical device 310 may preferably be carried on, e.g., a catheter 320 as seen in FIG. 4.

The apparatus of FIG. 4 is adapted to magnetize a medical device during the implantation process. To do so, the apparatus preferably includes a magnetic field generator 360 located proximate the proximal end 352 of the body 350. It may be preferred that the magnetic field generator 360 be positioned such that it does not enter the subject's body as does the distal end 354 of the body 350. During advancement through the body 350, it is preferred that the medical device 310 pass by the magnetic field generator 360 or through an opening formed within the magnetic field generator 360 such that a magnetic field is induced in the medical device 310.

In some embodiments, the magnetic field generator 360 may be in the form of one or more magnets. In other embodiments, the magnetic field generator 360 may be in the form of one or more electric coils connected to a power source 364 by a lead 362. In either form, the magnetic field generator 360 is preferably capable of inducing magnetization in magnetizable materials located in close proximity to the magnetic field generator 360.

Although the coating of medical devices with magnetic cells may be accomplished *in vitro,* it may be preferred that the medical devices be magnetically coated after placement in a desired location within the body, i.e., *in vivo.* To provide sufficient numbers of magnetic cells to adequately coat the magnetic contact surfaces of a medical device, however, it may be desirable to provide the medical device within a defined volume *in vivo.* A carrier liquid containing the selected magnetic cells can then be introduced into the defined volume, with the magnetic cells being magnetically attracted to and retained on the magnetic contact surfaces of the medical device. The defined volume may be provided in a manner that excludes other bodily fluids, such as, e.g., blood, cerebrospinal fluid, bile, etc. Such *in vivo* magnetic coating may preferably be accomplished after magnetization of the implanted medical device before implantation, during implantation, and/or after implantation as described herein.

In some instances, the defined volume into which the carrier liquid is introduced may be naturally occurring fluids such as, e.g., blood, bile, saliva, urine, cerebrospinal fluid and fluids in the gastrointestinal tract, peritoneal cavity, pleural space, pericardial sac and synovial fluid in joint cavities, etc.

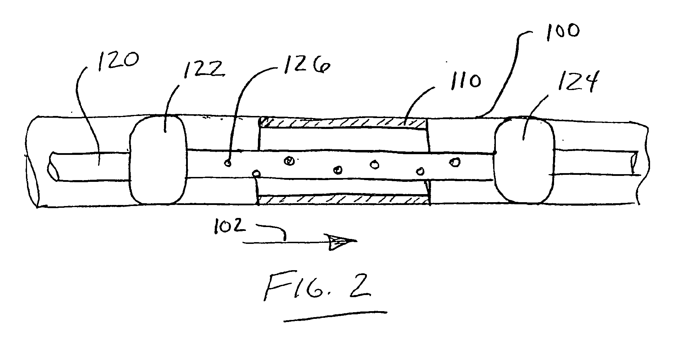

In other instances, it may be preferable to create the defined volume *in vivo.* FIG. 2 is a schematic diagram of one technique that may be used to create a defined volume *in vivo* into which the carrier liquid containing magnetic cells may be introduced. The depicted technique may be performed in a lumen 100 such as, e.g., blood vessel, using a catheter 120 that can be guided into position by any suitable technique, e.g., using a guidewire.

In the depicted embodiment, a stent 110 is in position within the vessel 100 before the catheter 120 is positioned therein. Alternatively, the catheter 120 may be used to deliver and deploy the stent 110 if, e.g., the catheter 120 includes a deployment balloon or other structure for deploying the stent 110 in the vessel 100.

With the stent 110 in position, the catheter 120 is positioned such that a first seal 122 is located upstream from the stent 110 and a second seal 124 is located downstream from the stent 110. In the depicted embodiment, upstream and downstream positions are relative to arrow 102 which is indicative of blood flow through the vessel 100. Once in position, the seals 122 and 124 are deployed to enclose a defined volume between the proximal seal 122 and the distal seal 124. The seals 122 and 124 may be, e.g., balloons that can be inflated through one or more lumens in the catheter 120.

With the seals 122 and 124 in operation, a carrier liquid with magnetic cells located therein can be delivered to the defined volume located in vessel 100 between seals 122 and 124. In the depicted embodiment, the carrier liquid with magnetic cells can be delivered through a lumen located in catheter 120, the carrier liquid and magnetic cells entering the defined volume through openings 126 in the catheter 120. Many other fluid delivery structures will be known to those skilled in the art.

If vessel 100 is a blood vessel and it is desirable to allow the carrier liquid and magnetic cells reside within the defined volume enclosed by seals 122 and 124 for a period of time longer than the flow of blood through vessel 100 can be terminated, the catheter 120 may be a perfusion catheter, i.e., a catheter that includes a lumen capable of passing blood from the upstream side of the first seal 122 to a point past the downstream side of the second seal 124.

After the carrier liquid and magnetic cells have resided in the defined volume for a sufficient time to magnetically coat the stent 110 with the magnetic cells, the seals 122 and 124 can be opened (e.g., deflated if they are provided in the form of balloons) such that stent 110 is no longer contained within a defined volume. If desired, the carrier liquid may be removed from the defined volume before the seals are opened.

In one variation of the device and method described with respect to FIG. 2, it should be understood that the first seal 122 may be optional. In other words, only the second seal 124 may be needed if the vessel 100 is used for the passage of a fluid such as blood on a continuous basis. Rather, by closing the downstream seal 124 and providing carrier liquid containing the desired magnetic cells at a pressure greater than, e.g., the blood pressure at the location of the stent 110, the carrier liquid and magnetic cells may be entrapped within the volume defined by the downstream seal 124 and the fluid (e.g., blood) flowing against the seal 124. In some instances, it may be possible to deliver the carrier liquid on the upstream side of the seal 124 using, e.g., the lumen of guide catheter used to deliver the device depicted in FIG. 2. In some instances it may be possible to deliver the carrier liquid downstream of seal 122 (e.g., without deploying seal 124 or using a device that includes only one seal). The lack of blood flow caused by occlusion due to seal 122 may preferably allow the delivered liquid to remain downstream of seal 122 for period of time sufficient to magnetically coat the stent 110.

Examples of devices capable of operating as described in connection with FIG. 2 may be found in, e.g., U.S. Patent Nos. 6,575,932 (OBrien et al.); 5,558,642 (Schweich, Jr. et al.); 5,135,484 (Wright), etc.

In some instances, it may be possible, or even desirable to magnetically coat a magnetic medical device *in vitro.* For example, the present disclosure may be in the form of a kit that includes a flexible tubular conduit designed for placement as, e.g., a synthetic blood vessel or an arterio-veinous graft for dialysis. Such a conduit may preferably include a lumen defined by natural or synthetic biocompatible fibrous fabrics with magnetized filaments woven into the fabric to attract and retain magnetic cells to the interior surfaces of the lumen. Such a conduit may be filled with a carrier liquid and magnetic cells *in vitro*. The *in vitro* residence time of the carrier liquid may be extended until the magnetic cells are established on the interior surfaces of the lumen, after which the conduit may be surgically implanted as, e.g., a bypass graft or an arterio-veinous fistula.

### KITS

The various components and materials described herein may be assembled into different kits to assist practitioners in practicing the methods of the present disclosure. These kits may preferably be contained within a single, sterilizable package to further enhance the ease of use by practitioners. Some exemplary embodiments of such kits may be described below.

Such a kit may include, e.g., an implantable medical device with a magnetic contact surface and means for enclosing a defined volume *in vivo* in which the implantable medical device is located when implanted. The means for enclosing a defined volume may include any of the structures described herein and equivalents thereof. The implantable medical device in the kit may be selected from, e.g., the group consisting of a blood vessel stent, vascular graft, prosthesis for any biologic conduit; heart valve, artificial heart, left ventricular assist device, and electronic leads. The means for enclosing a defined volume *in vivo* may be adapted for enclosing the defined volume in a biologic conduit such as, e.g., a blood vessel.

Such a kit may include, e.g., an implantable medical device with a magnetic contact surface and a catheter for creating a defined volume in a biologic conduit *in vivo.* The catheter may include at least one seal capable of closing the biologic conduit when deployed within the conduit, a carrier liquid lumen extending from a proximal end of the catheter to a location proximate the at least one seal, and an opening in the carrier liquid lumen proximate the at least one seal, wherein carrier liquid in the carrier liquid lumen is capable of exiting the catheter proximate the seal such that the carrier liquid is located within the defined volume. In such a kit, the at least one seal may include a pair of seals such that the defined volume is enclosed by the pair of seals when the pair of seals are deployed within the biologic conduit. Each seal may include an inflatable balloon, and the catheter may include one or more inflation lumens extending from the proximal end of the catheter to the seals. The components of the kit may be adapted for deployment within a biologic conduit in the form of blood vessel.

Another embodiment of a kit that may be provided is a kit for magnetizing a medical device *in vivo.* The kit may include an implantable medical device comprising magnetic material and a magnetizing apparatus for magnetizing the magnetic material of the implantable medical device *in vivo.* The magnetizing apparatus may include a medical grade catheter with a proximal end and a distal end. A magnetic field generator may be operably attached to the catheter, wherein the magnetic field generator is located proximate the distal end of the catheter. The magnetic field generator may preferably be sized such that it can be advanced through a biologic conduit of a subject. The implantable medical device may be selected from the group consisting of a blood vessel stent, vascular graft, prosthesis for any biologic conduit; heart valve, artificial heart, left ventricular assist device, and electronic leads. The magnetic field generator may include a permanent magnet. Alternatively, the magnetic field generator may include an electric coil and an electrical lead attached to the electric coil, the electrical lead extending towards the proximal end of the catheter, whereby electrical energy can be delivered to the electric coil through the electrical lead. In some embodiments, the electrical lead may extend to the proximal end of the catheter. The kit may also include a power source for delivering electrical energy to the electric coil. In still other embodiments, the kit may include (as described herein) means for enclosing a defined volume *in vivo* in which the implantable medical device is located when implanted.

Still another embodiment of a kit according to the present disclosure may include a kit for magnetizing a medical device, wherein the kit includes an implantable medical device comprising magnetic material and a magnetizing apparatus for magnetizing an implantable medical device. The magnetizing apparatus may include an elongated body with a proximal end and a distal end, wherein the elongated body includes a lumen extending from the proximal end to the distal end. The magnetizing apparatus may also include a magnetic field generator located proximate the proximal end of the elongated body, wherein a medical device advanced through the lumen from the proximal end towards the distal end passes through a magnetic field generated by the magnetic field generator. The magnetic field generator may include a permanent magnet. In some embodiments, the magnetic field generator includes an electric coil and an electrical lead attached to the electric coil, whereby electrical energy can be delivered to the electric coil through the electrical lead. The kit may also include a power source for delivering electrical energy to the electric coil. The elongated body may preferably be in the form of a guide catheter adapted for insertion into a biologic conduit such as, e.g., a blood vessel. The elongated body may alternatively be in the form of an introducer sheath.

The various kits may also include means for associating a magnetic charge with a plurality of biologic cells as described herein. The kit may also include a carrier liquid for delivery to the enclosed defined volume *in vivo.* The carrier liquid may preferably be a biologically compatible liquid such as, e.g., 0.9% normal saline, Ringer's solution, tissue culture media, serum, and plasma. The kit may also include means for delivering the carrier liquid to the enclosed defined volume *in vivo,* such as, e.g., a catheter, a lumen in a catheter, syringe, needle, etc.

## Claims

1. A kit for magnetically coating a medical device *in vivo,* the kit comprising:
an implantable medical device comprising a magnetic contact surface; and
a catheter for creating a defined volume in a biologic conduit *in vivo,* wherein the catheter comprises:
at least one seal capable of closing the biologic conduit when deployed within the conduit;
a carrier liquid lumen extending from a proximal end of the catheter to a location proximate the at least one seal;
an opening in the carrier liquid lumen proximate the at least one seal, wherein carrier liquid in the carrier liquid lumen is capable of exiting the catheter proximate the seal.

2. A kit according to claim 1, wherein the at least one seal comprises a pair of seals such that the defined volume is enclosed by the pair of seals when the pair of seals are deployed within the biologic conduit.

3. A kit according to claim 1, wherein each seal comprises an inflatable balloon, and further wherein the catheter comprises one or more inflation lumens extending from the proximal end of the catheter to the seals.

4. A kit according to claim 1, wherein the biologic conduit comprises a blood vessel.

5. A kit according to claim 1, further comprising means for associating a magnetic charge with a plurality of biologic cells.

6. A kit according to claim 1, further comprising a carrier liquid for delivery to the defined volume *in vivo.*

7. A kit according to claim 6, wherein the carrier liquid is a biologically compatible liquid.

8. A kit according to claim 6, further comprising means for delivering the carrier liquid to the defined volume *in vivo.*

9. A kit according to claim 8, wherein the means for delivering comprises the carrier liquid lumen of the catheter.

## Patentansprüche

1. Set zum magnetischen Beschichten eines Medizinprodukts in vivo, wobei das Set aufweist:
ein implantierbares Medizinprodukt mit einer magnetischen Kontaktfläche; und
einen Katheter zum Erzeugen eines definierten Volumens in einem biologischen Kanal in vivo, wobei der Katheter aufweist:
mindestens eine Dichtung, die den biologischen Kanal verschließen kann, wenn sie im Kanal eingesetzt ist;
ein Trägerflüssigkeitslumen, das sich von einem proximalen Ende des Katheters zu einer Stelle nahe der mindestens einen Dichtung erstreckt;
eine Öffnung im Trägerflüssigkeitslumen nahe der mindestens einen Dichtung, wobei Trägerflüssigkeit im Trägerflüssigkeitslumen aus dem Katheter nahe der Dichtung austreten kann.

2. Set nach Anspruch 1, wobei die mindestens eine Dichtung ein Paar Dichtungen so aufweist, dass das definierte Volumen durch das Paar Dichtungen eingeschlossen ist, wenn das Paar Dichtungen im biologischen Kanal eingesetzt ist.

3. Set nach Anspruch 1, wobei jede Dichtung einen inflatierbaren Ballon aufweist, und wobei ferner der Katheter ein oder mehrere Inflationslumen aufweist, die sich vom proximalen Ende des Katheters zu den Dichtungen erstrecken.

4. Set nach Anspruch 1, wobei der biologische Kanal ein Blutgefäß aufweist.

5. Set nach Anspruch 1, ferner mit einer Einrichtung zum Assoziieren einer magnetischen Ladung mit mehreren biologischen Zellen.

6. Set nach Anspruch 1, ferner mit einer Trägerflüssigkeit zur Abgabe zum definierten Volumen in vivo.

7. Set nach Anspruch 6, wobei die Trägerflüssigkeit eine biologisch kompatible Flüssigkeit ist.

8. Set nach Anspruch 6, ferner mit einer Einrichtung zum Abgeben der Trägerflüssigkeit zum definierten Volumen in vivo.

9. Set nach Anspruch 8, wobei die Einrichtung zum Abgeben das Trägerflüssigkeitslumen des Katheters aufweist.

## Revendications

1. Kit pour recouvrir magnétiquement un dispositif médical in vivo, le kit comprenant :
un dispositif médical implantable comprenant une surface de contact magnétique ; et
un cathéter pour créer un volume défini dans un conduit biologique in vivo, dans lequel le cathéter comprend :
au moins un joint d'étanchéité pouvant fermer le conduit biologique lorsqu'il est déployé à l'intérieur du conduit ;
une lumière de liquide porteur s'étendant à partir d'une extrémité proximale du cathéter jusqu'à un emplacement à proximité de l'au moins un joint d'étanchéité ;
une ouverture dans la lumière de liquide porteur à proximité de l'au moins un joint d'étanchéité, dans lequel du liquide porteur dans la lumière de liquide porteur peut sortir du cathéter à proximité du joint d'étanchéité.

2. Kit selon la revendication 1, dans lequel l'au moins un joint d'étanchéité comprend une paire de joints d'étanchéité de sorte que le volume défini est enfermé par la paire de joints d'étanchéité lorsque la paire de joints d'étanchéité est déployée à l'intérieur du conduit biologique.

3. Kit selon la revendication 1, dans lequel chaque joint d'étanchéité comprend un ballonnet gonflable, et en outre dans lequel le cathéter comprend une ou plusieurs lumières de gonflage s'étendant à partir de l'extrémité proximale du cathéter jusqu'aux joints d'étanchéité.

4. Kit selon la revendication 1, dans lequel le conduit biologique comprend un vaisseau sanguin.

5. Kit selon la revendication 1, comprenant en outre des moyens pour associer une charge magnétique à une pluralité de cellules biologiques.

6. Kit selon la revendication 1, comprenant en outre un liquide porteur pour la distribution au volume défini in vivo.

7. Kit selon la revendication 6, dans lequel le liquide porteur est un liquide biologiquement compatible.

8. Kit selon la revendication 6, comprenant en outre des moyens pour distribuer le liquide porteur au volume défini in vivo.

9. Kit selon la revendication 8, dans lequel les moyens de distribution comprennent la lumière de liquide porteur du cathéter.
